# DEMANDE DE BREVET EUROPEEN

(11) **EP 1 348 703 A1**
(43) Date de publication de la demande: **01.10.2003**
(21) Numéro de dépôt: 03290636.4
(22) Date de dépôt: 13.03.2003
(51) Int. Cl.: C07D 317/60, A61K 31/36, A61P 9/12

(54) **Formes polymorphes du fasidotril, leurs procédés de préparation et compositions pharmaceutiques les contenant**

(30) Priorité: 29.03.2002 FR 0204036
(71) Demandeur: BIOPROJET, 75003 Paris (FR)
(72) Inventeur: Capet, Marc, 35520 Melesse (FR); Coquerel, Gérard, 76520 Boos (FR); Danvy, Denis, 76190 Yvetot (FR); Lecomte, Jeanne-Marie, 75003 Paris (FR); Petit, Marie-Noelle, 76130 Mont Saint-Aignan (FR); Schwartz, Jean-Charles, 75014 Paris (FR)
(74) Mandataire: Bernasconi, Jean

(57) **Abrégé**

La présente invention a pour objet les différentes formes polymorphes I, II, III, IV du (S,S)-2-(2-acétylsulfanylméthyl-3-benzo[1,3]dioxol-5-ylpropionylamino) propionate de benzyle, ci-après appelé Fasidotril, leurs procédés de préparation et les nouvelles compositions pharmaceutiques qui les contiennent.

## Description

La présente invention a pour objet les différentes formes polymorphes du (S,S)-2-(2-acétylsulfanylméthyl-3-benzo[1,3]dioxol-5-ylpropionylamino)propionate de benzyle, ci-après appelé Fasidotril, et les méthodes de préparation de celles-ci.

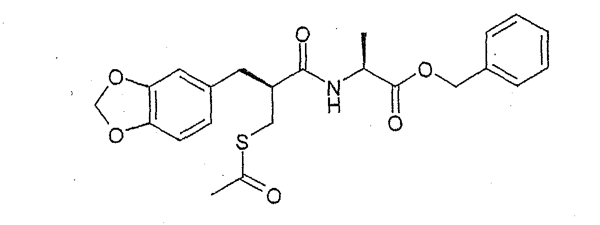

Le Fasidotril, (S,S)-2-(2-acétylsulfanylméthyl-3-benzo[1,3]dioxol-5-ylpropionyl amino)propionate de benzyle, peut être préparé par application des méthodes décrites dans le brevet EP 0.419.327 B1 ou le brevet FR 0014419 déposés par la Société Civile Bioprojet et présente des propriétés intéressantes en tant qu'inhibiteur mixte des enzymes enképhalinase (EC 3.4.24.11) et enzyme de conversion de l'angiotensine (EC 3.4.15.1 ; ACE). On sait en effet que l'enképhalinase est une peptidase dégradant notamment les enképhalines. La méthionine et la leucine enképhaline sont des peptides qui ont été découverts dans le cerveau et qui sont des ligands endogènes du récepteur morphinique. Par ailleurs, le facteur auriculaire natriurétique (ANF) est un peptide endogène exerçant des effets vasorelaxant, diurétique et natriurétique potentiellement bénéfiques dans le traitement d'affections cardiovasculaires et rénales. L'ANF est un substrat de l'enképhalinase et les inhibiteurs de cette peptidase ralentissent sa dégradation, augmentent son taux plasmatique et induisent des effet antihypertenseur, diurétique et natriurétique (Lecomte et al., Proc. Natl. Ac. Sci. USA1989, 86:*19*:7580-4).

On sait également, par exemple par le brevet français N°2.623.498 déposé au nom de la demanderesse, que certains dérivés d'aminoacides exercent une activité inhibitrice sur l'enzyme de conversion de l'angiotensine I en angiotensine II (ACE), l'angiotensine II étant une substance vasomotrice active considérée comme l'agent responsable de diverses formes de l'hypertension. Ces composés sont donc utiles pour le traitement de l'hypertension et de l'insuffisance cardiaque.

Il a été découvert que le Fasidotril peut exister sous différentes formes cristallines polymorphes, qui différent les unes des autres par leurs propriétés physiques, spectroscopiques, leurs zones de stabilité thermodynamique et leurs méthodes de préparation. Quatre de ces nouvelles formes sont décrites ci-après et seront désignées respectivement comme étant forme I, forme II, forme III et forme IV.

Il est montré que parmi ces quatre formes, les formes I et II sont stables respectivement à basse et haute température alors que les formes III et IV sont instables et peuvent aisément être converties en forme stable I ou II selon les conditions opératoires choisies.

Il est aussi montré qu'il est possible de transformer la forme I en forme II ou de transformer la forme II en forme I selon les conditions opératoires choisies.

Les différentes formes polymorphes du Fasidotril peuvent être préparées par cristallisation, à une température comprise entre la température de congélation de la solution et la température d'ébullition du mélange, d'une solution du Fasidotril dans un solvant choisi parmi les hydrocarbures saturés (par exemple pentane, cyclohexane) ou insaturés (par exemple toluène, xylène), les alcools (par exemple méthanol, éthanol, 2-propanol), les éthers (par exemple oxyde de diisopropyle, oxyde de diéthyle), les esters (par exemple acétate d'éthyle, acétate d'isopropyle), les amides (par exemple diméthylformamide, N-méthylpyrrolidone, diméthylacétamide), les nitriles (par exemple acétonitrile), les halogénés (par exemple dichlorométhane, dichloroéthane, chlorobenzène), l'eau ou un mélange de ces solvants. Cette cristallisation peut être spontanée ou provoquée par refroidissement lent ou rapide, ajout d'un solvant, d'amorces constituées de Fasidotril cristallisé ou d'un autre produit solide, évaporation ou distillation du solvant. De plus, la solution initiale de Fasidotril peut contenir d'autres produits, par exemple un isomère du Fasidotril.

Dans le cas, par exemple de l'obtention par cristallisation spontanée, on peut obtenir la forme I en choisissant une température inférieure à 25°, ou la forme II en choisissant une température supérieure, par exemple 50°C et en maintenant cette température pendant une durée suffisante.

Les différentes formes polymorphes stables du Fasidotril peuvent aussi être préparées par transformation d'une forme stable ou métastable dans une autre. Cette transformation peut être réalisée en solution ou en suspension en ensemençant éventuellement avec une forme du Fasidotril solide. Cette opération s'effectue à une température comprise entre la température de congélation du solvant et sa température d'ébullition, dans un solvant choisi parmi les hydrocarbures saturés (par exemple pentane, cyclohexane) ou insaturés (par exemple toluène, xylène), les alcools (par exemple méthanol, éthanol, isopropanol), les éthers (par exemple oxyde de diisopropyle, oxyde de diéthyle), les esters (par exemple acétate d'éthyle, acétate d'isopropyle), les amides (par exemple diméthylformamide, N-méthylpyrrolidone, diméthylacétamide), les nitriles (par exemple acétonitrile), l'eau ou un mélange de ces solvants.

Les différentes formes polymorphes du Fasidotril peuvent être caractérisées par diffraction des rayons X (voir Fig. 4), par calorimétrie différentielle à balayage, par infrarouge, par RMN du solide ou par toute autre méthode connue de l'homme de l'art.

La forme I présente un spectre de diffraction des rayons X sur poudre original (mesure réalisée sur un diffractomètre Siemens D5005 avec une anticathode au cuivre ; exploitation des résultats réalisée avec le logiciel Eva v 7.0) avec les raies caractéristiques suivantes (2théta en °) :
17,1 ; 17,6 ; 17,9 ; 18,1 ; 18,8 ; 19,5 ; 20,0 ; 21,3 ; 22,1 ; 23,3 ; 24,8 ; 25,0 ; 27,8

Le spectre de calorimétrie différentielle à balayage à 2°C/min montre deux maxima, l'un à 110,3°C, l'autre à 114,1°C avec des enthalpies de 40,13 J/g et 65,47 J/g respectivement et un onset de fusion à 108,8°C.

Le spectre infrarouge en comprimé KBr présente les absorptions caractéristiques aux longueurs d'ondes suivantes (en cm-1, f pour faible, m pour moyen, F pour fort) :
3280 (F), 3080 (f), 3040 (f), 3000 (f), 2920 (m), 2800 (f), 1730 (F), 1690 (F), 1680 (F), 1640 (F), 1610 (f), 1540 (F), 1500 (F), 1480 (F), 1440 (F), 1400 (f), 1380 (m), 1360 (f), 1330 (m), 1310 (m), 1280 (m), 1260 (F), 1250 (F), 1220 (F), 1200 (F) , 1130 (F), 1110 (F), 1060 (f), 1040 (F), 1010 (f), 1000 (f), 960 (F), 940 (f), 930 (m), 900 (m), 860 (f), 810 (m), 790 (m), 750 (F), 720 (f), 700 (F), 680 (f), 620 (F), 600 (f), 580 (f) , 540 (f) , 520 (f), 480 (f), 460 (f), 450 (f), 430 (f)

La forme II présente un spectre de diffraction des rayons X sur poudre original (mesure réalisée sur un diffractomètre Siemens D5005 avec une anticathode au cuivre ; exploitation des résultats réalisée avec le logiciel Eva v 7.0) avec les raies caractéristiques suivantes (2théta en °) :
8,1 ; 12,9 ; 16,2 ; 16,8 ; 17,3 ; 17,8 ; 18,4 ; 20,8 ; 23,8 ; 27,7

Le spectre de calorimétrie différentielle à balayage à 2°C/min montre un maximum à 114,8°C avec une enthalpie de 101,64 J/g et un onset de fusion à 112,6°C.

Le spectre infrarouge en comprimé KBr présente les absorptions caractéristiques aux longueurs d'ondes suivantes (en cm-1, f pour faible, m pour moyen, F pour fort) :
3320 (F), 3280 (F), 3080 (f) , 3000 (f) , 2920 (m), 2880 (m), 2800 (f), 1730 (F), 1690 (F), 1680 (F), 1640 (F), 1540 (F), 1500 (F), 1480 (F), 1440 (F), 1400 (f), 1380 (m), 1360 (m), 1330 (m), 1310 (m), 1280 (m), 1260 (F), 1220 (F), 1200 (F), 1160 (f), 1130 (F), 1110 (F), 1060 (f), 1040 (F), 1010 (f), 1000 (f), 960 (F), 940 (m), 930 (m), 900 (m), 860 (f), 810 (m), 790 (m), 750 (F) , 700 (F), 620 (F), 600 (f), 580 (f), 540 (f), 520 (f), 480 (f), 460 (f), 430 (f).

La forme III présente un spectre de diffraction des rayons X sur poudre original (mesure réalisée sur un diffractomètre Siemens D5005 avec une anticathode au cuivre ; exploitation des résultats réalisée avec le logiciel Eva v 7.0) avec les raies caractéristiques suivantes (2théta en °) :
11,1 ; 12,2 ; 16,4 ; 16,7 ; 17,7 ; 17,9 ; 18,6 ; 19,1 ; 20,2 ; 20,6 ; 21,3 ; 22,2 ; 22,6 ; 24,2 ; 24,7 ; 26,8 ; 28,6

Le spectre de calorimétrie différentielle à balayage à 0,2°C/min montre quatre maxima à 101,3°C, 103,6°C, 105,9°C et 113,9°C avec des enthalpies de 3,62 J/g, 3,92 J/g, 1,61 J/g et 94,08 J/g respectivement et des onsets à 100,7°C, 103,0°C, 104,8°C et 112,1°C respectivement.

La forme IV présente un spectre de diffraction des rayons X sur poudre original (mesure réalisée sur un diffractomètre Siemens D5005 avec une anticathode au cuivre ; exploitation des résultats réalisée avec le logiciel Eva v 7.0) avec les raies caractéristiques suivantes (2théta en °) :
8,8 ; 16,0 ; 17,2 ; 17,7 ; 18,4 ; 19,1 ; 19,3 ; 20,3 ; 21,3 ; 22,9 ; 25,1 ; 25,5 ; 26,5 ; 28,8

Le spectre de calorimétrie différentielle à balayage à 0,2°C/min montre trois maxima à 90,4°C, 103,0°C et 113,5°C avec des enthalpies de 1,32 J/g, 12,91 J/g et 96,10 J/g respectivement avec des onsets à 87,0°C, 101,5°C et 111,2°C respectivement.

La présente invention concerne également les compositions pharmaceutiques qui contiennent, à titre de principe actif, le Fasidotril sous l'une de ses formes polymorphes.

Ces compositions sont administrables à l'homme par voie orale, parentérale ou rectale.

Ces compositions pharmaceutiques peuvent être sous forme solide ou liquide et se présenter sous les formes pharmaceutiques couramment utilisées en médecine humaine comme, par exemple, sous forme de comprimés simples ou dragéifiés, de gélules, de suppositoires, de préparations injectables.

Les compositions pharmaceutiques conformes à l'invention, sont administrables à des doses unitaires, de préférence de 1 à 200 mg de principe actif et à une posologie quotidienne pouvant varier de 2 à 400 mg de principe actif.

De façon avantageuse une composition pharmaceutique peut comprendre la forme stable I ou II, mais on peut également préparer des compositions pharmaceutiques avec les formes III ou IV.

L'invention a également pour objet un procédé de traitement d'un patient, qui en a besoin, par l'inhibition de l'enképhalinase et de l'enzyme de conversion de l'angiotensine.

Elle a notamment trait à un procédé de traitement de l'hypertension et/ou de l'insuffisance cardiaque dans lequel on administre au patient une composition pharmaceutique comprenant l'une des formes I, II, III ou IV du Fasidotril.

De préférence, et à moins que l'on n'utilise une forme retard, ce procédé prévoit l'administration d'une posologie quotidienne comprise entre 2 et 400 mg de Fasidotril, de. préférence administré sous forme de doses unitaires de 1 à 200 mg.

L'invention a également pour objet l'utilisation de l'une des formes du I à IV pour préparer une composition pharmaceutique telle que décrite ci-dessus pour le traitement de l'hypertension et/ou de l'insuffisance cardiaque.

Les exemples donnés ci-après illustrent la présente invention sans pour autant la limiter.

Dans les exemples suivants, le Fasidotril est synthétisé par l'un quelconque des procédés adaptés au Fasidotril et décrits dans les brevets EP 419 327 ou FR 0014419. Les puretés des formes polymorphiques obtenues après mise en oeuvre des exemples suivants sont supérieures à 95% (limite de précision de la technique d'analyse utilisée).

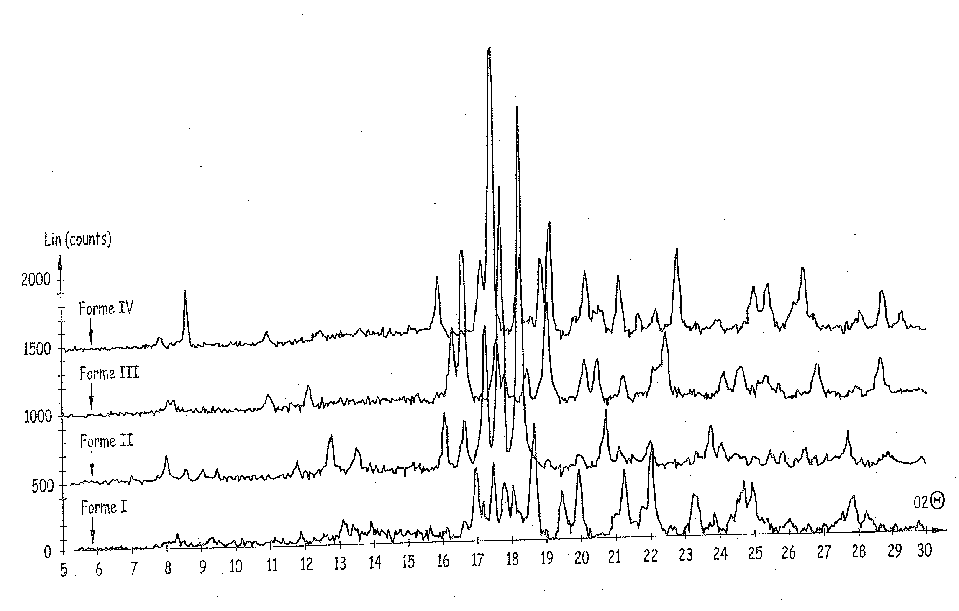

La figure 1 représente les spectres de diffraction sur poudre des formes I, II, III et IV.

### Exemple 1 : Fasidotril forme III

Un ballon contenant 1 g de Fasidotril est chauffé jusqu'à fusion du produit. Le ballon est rapidement refroidi par plongée dans un bain glace-eau. On obtient ainsi 1 g de Fasidotril dont le spectre de diffraction sur poudre est conforme à celui de la forme III.

### Exemple 2 : Fasidotril forme IV

La dissolution de 1 g de Fasidotril dans 15 mL de 2-propanol est effectuée par chauffage à une température voisine de 70°C. A cette solution sont ajoutés 20 mL d'heptane refroidi à -70°C. La température chute à 25°C et une cristallisation se produit rapidement. Le milieu est agité encore 4 minutes, filtré et séché sous pression réduite (20 mm Hg) à 25°C. On obtient ainsi 0,77 g de Fasidotril dont le spectre de diffraction sur poudre est conforme à celui de la forme IV.

### Exemple 3 : Fasidotril forme IV

Un ballon contenant une solution de 2 g de Fasidotril dans 35 mL de 2-propanol portée à une température de 80°C, est brutalement plongé dans un bain d'acétone saturée en carboglace. La précipitation est immédiate. Après 2 minutes d'agitation, la suspension est filtrée et séchée sous pression réduite (20 mm Hg) à 25°C. On obtient ainsi 1,75 g de Fasidotril dont le spectre de diffraction sur poudre est conforme à celui de la forme IV.

### Exemple 4 : Fasidotril forme II

Dans une étuve ventilée à 94°C, un flacon en verre bouché contenant 0,2 g de Fasidotril forme IV est laissé pendant 3 jours. On obtient ainsi 0,2 g de Fasidotril dont le spectre de diffraction sur poudre est conforme à celui de la forme II.

### Exemple 5 : Fasidotril forme III

Un sublimateur d'un diamètre intérieur de 45 mm contenant 2 g de Fasidotril est chauffé sous vide à une pression de 0,1 mm de Hg. Après arrêt du chauffage et retour à la pression atmosphérique, le solide déposé sur le réfrigérant est récupéré. On obtient ainsi 0,4 g de Fasidotril dont le spectre de diffraction sur poudre est conforme à celui de la forme III.

### Exemple 6 : Fasidotril forme I

La dissolution de 5g de Fasidotril dans 20 ml d'acétone est effectuée par chauffage à une température voisine de 50°C. Le chauffage est arrêté et la solution ainsi obtenue est laissée à refroidir. La cristallisation se produit à 32°C. L'agitation est maintenue et la suspension est refroidie à -5°C. Le solide est séparé par filtration et séché sous pression réduite (20 mm Hg) à 20°C. On obtient ainsi 4,66g de Fasidotril dont le spectre de diffraction de poudre est conforme à celui de la forme I.

### Exemple 7 : Fasidotril forme I

La dissolution de 30g de Fasidotril dans 120 ml d'acétate d'éthyle est effectuée par chauffage à une température voisine de 52°C. Le chauffage est arrêté et la solution ainsi obtenue est refroidie à l'aide d'un bain d'eau à 10°C. Une cristallisation rapide se produit à 31°C. La suspension est maintenue, sous agitation, à 30°C pendant 5 minutes, puis est refroidie à 0°C. Le solide est séparé par filtration, rincé par 20 ml d'acétate d'éthyle à 5°C et séché sous pression réduite (20 mm Hg) à 20°C. On obtient ainsi 25,40g de Fasidotril dont le spectre de diffraction de poudre est conforme à celui de la forme I.

### Exemple 8 : Fasidotril forme I

A une suspension de 1g de Fasidotril forme IV dans 20 ml d'un mélange dichlorométhane/ 2-propanol (2/18 en volumes), on ajoute 2 mg de Fasidotril forme II. Le mélange est agité pendant onze jours à 20°C. L'insoluble est séparé par filtration et séché sous vide pour conduire à 0,6g de Fasidotril dont le spectre de diffraction de poudre est conforme à celui de la forme I.

### Exemple 9 : Fasidotril forme I

A une suspension de 1g de Fasidotril forme III dans 20 ml d'un mélange dichlorométhane / 2-propanol (2/18 en volumes), on ajoute 2 mg de Fasidotril forme IV. Le mélange est agité pendant douze jours à 20°C. L'insoluble est séparé par filtration et séché sous vide pour conduire à 0,5g de Fasidotril dont le spectre de diffraction de poudre est conforme à celui de la forme I.

### Exemple 10: Fasidotril forme I

La dissolution de 13,88 g de Fasidotril dans 207 mL de 2-propanol est obtenue par chauffage à une température voisine de 70°C. La solution ainsi obtenue est refroidie à une température voisine de 50°C et la cristallisation amorcée par introduction de quelques cristaux de Fasidotril forme I. La suspension est refroidie en deux heures jusqu'à une température voisine de 35°C, puis est maintenue à cette température pendant une heure et demie. Le solide est séparé par filtration, rincé par 15 mL de 2-propanol et séché sous pression réduite (20 mm Hg) à 25°C. On obtient ainsi 11,16 g de Fasidotril dont le spectre de diffraction sur poudre est conforme à celui de la forme I.

### Exemple 11 : Fasidotril forme I

A une suspension de 1 g de Fasidotril forme IV dans 20 mL d'un mélange dichlorométhane / 2-propanol (2/18 en volumes), on ajoute 2 mg de Fasidotril forme I. Le mélange est agité pendant douze jours à 20°C. L'insoluble est séparé par filtration et séché sous vide pour conduire à 0,34 g de Fasidotril dont le spectre de diffraction sur poudre est conforme à celui de la forme I.

### Exemple 12 : Fasidotril forme II

A une suspension de 1 g de Fasidotril forme I dans 20 mL de 2-propanol, on ajoute 2 mg de Fasidotril forme II. Le mélange est agité pendant cinq jours à 45°C. L'insoluble est séparé par filtration, et séché sous vide pour conduire à 0,58 g de Fasidotril dont le spectre de diffraction sur poudre est conforme à celui de la forme II.

### Exemple 13 : Fasidotril forme II

A une suspension de 0,5 g de Fasidotril forme I dans 10 mL de 2-propanol, on ajoute 2 mg de Fasidotril forme III. Le mélange est agité pendant quatre jours à 45°C. L'insoluble est séparé par filtration et séché sous vide pour conduire à 0,17 g de Fasidotril dont le spectre de diffraction sur poudre est conforme à celui de la forme II.

### Exemple 14 : Fasidotril forme I

A une suspension de 1 g de Fasidotril forme II dans 20 mL d'un mélange dichlorométhane / 2-propanol (4/16 en volumes), on ajoute 2 mg de Fasidotril forme I. Le mélange est agité pendant quatorze jours à 20°C. L'insoluble est séparé par filtration et séché sous vide pour conduire à 0,6 g de Fasidotril dont le spectre de diffraction sur poudre est conforme à celui de la forme I.

### Exemple 15 : Fasidotril forme I

A une suspension de 1 g de Fasidotril forme III dans 20 mL d'un mélange dichlorométhane / 2-propanol (2/18 en volumes), on ajoute 2 mg de Fasidotril forme I. Le mélange est agité pendant douze jours à 20°C. L'insoluble est séparé par filtration et séché sous vide pour conduire à 0,51 g de Fasidotril dont le spectre de diffraction sur poudre est conforme à celui de la forme I.

## Revendications

1. Formes polymorphes du (S,S)-2-(2-acétylsulfanylméthyl-3-benzo[1,3]dioxol-5-ylpropionylamino)propionate de benzyle 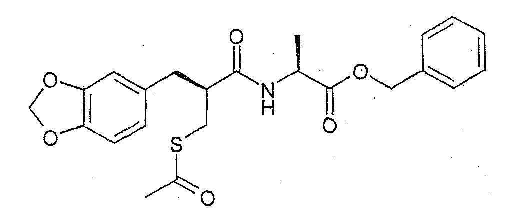 **caractérisées par** le spectre de diffraction des rayons X sur poudre (2théta en °)
forme I : 17,1 ; 17,6 ; 17,9 ; 18.1 ; 18,8 ; 19,5 ; 20,0 ; 21,3 ; 22,1 ; 23,3 ; 24,8 ; 25,0 ; 27,8
forme II : 8,1 ; 12,9 ; 16,2 ; 16,8 ; 17,3 ; 17,8 ; 18,4 ; 20,8 ; 23,8 ; 27,7
forme III : 11,1 ; 12,2 ; 16,4 ; 16,7 ; 17,7 ; 17,9 ; 18,6 ; 19,1 ; 20,2 ; 20,6 ; 21,3 ; 22,2 ; 22,6 ; 24,2 ; 24,7 ; 26,8 ; 28,6
forme IV : 8,8 ; 16,0 ; 17,2 ; 17,7 ; 18,4 ; 19,1 ; 19,3 ; 20,3 ; 21,3 ; 22,9 ; 25,1 ; 25,5 ; 26,5 ; 28,8
et leur spectre de calorimétrie différentielle à balayage forme I (2°C/min) : deux maxima, l'un à 110,3°C, l'autre à 114,1°C avec des enthalpies de 40,13 J/g et 65,47 J/g respectivement et un onset de fusion à 108,8°C
forme II (2°C/min) : maximum à 114,8°C avec une enthalpie de 101,64 J/g et un onset de fusion à 112,6°C
forme III (0,2°C/min) : quatre maxima à 101,3°C, 103,6°C, 105,9°C et 113,9°C avec des enthalpies de 3,62 J/g, 3,92 J/g, 1,61 J/g et 94,08 J/g respectivement et des onsets à 100,7°C, 103,0°C, 104,8°C et 112,1°C respectivement forme IV (0,2°C/min) : trois maxima à 90,4°C, 103,0°C et 113,5°C avec des enthalpies de 1,32 J/g, 12,91 J/g et 96,10 J/g respectivement avec des onsets à 87,0°C, 101,5°C et 111,2°C respectivement.

2. Forme polymorphe stable I du (S,S)-2-(2-acétylsulfanylméthyl-3-benzo[1,3]dioxol-5-ylpropionylamino) propionate de benzyle **caractérisées par** le spectre de diffraction des rayons X sur poudre (2théta en °)
17,1 ; 17,6 ; 17,9 ; 18,1 ; 18,8 ; 19,5 ; 20,0 ; 21,3 ; 22,1 ; 23,3 ; 24,8 ; 25,0 ; 27,8
le spectre de calorimétrie différentielle à balayage (2°C/min) : deux maxima, l'un à 110,3°C, l'autre à 114,1°C avec des enthalpies de 40,13 J/g et 65,47 J/g respectivement et un onset de fusion à 108,8°C
et le spectre infrarouge en comprimé KBr (absorption en cm-1) 3280 (F), 3080 (f), 3040 (f), 3000 (f), 2920 (m), 2800 (f), 1730 (F), 1690 (F), 1680 (F), 1640 (F), 1610 (f), 1540 (F), 1500 (F), 1480 (F), 1440 (F), 1400 (f), 1380 (m), 1360 (f), 1330 (m), 1310 (m), 1280 (m), 1260 (F), 1250 (F), 1220 (F), 1200 (F), 1130 (F), 1110 (F), 1060 (f), 1040 (F), 1010 (f), 1000 (f), 960 (F), 940 (f), 930 (m), 900 (m), 860 (f), 810 (m), 790 (m), 750 (F), 720 (f), 700 (F), 680 (f), 620 (F), 600 (f), 580 (f), 540 (f), 520 (f) , 480 (f), 460 (f), 450 (f), 430 (f).

3. Forme polymorphe stable II du (S,S)-2-(2-acétylsulfanylméthyl-3-benzo[1,3]dioxol-5-ylpropionylamino) propionate de benzyle **caractérisées par** le spectre de diffraction des rayons X sur poudre (2théta en °)
8,1 ; 12,9 ; 16,2 ; 16,8 ; 17,3 ; 17,8 ; 18,4 ;20,8 ; 23,8 ; 27,7
le spectre de calorimétrie différentielle à balayage
Forme II (2°C/min) : maximum à 114,8°C avec une enthalpie de 101,64 J/g et un onset de fusion à 112,6°C
et le spectre infrarouge en comprimé KBr (absorption en cm-1). 3320 (F), 3280 (F), 3080 (f), 3000 (f), 2920 (m), 2880 (m), 2800 (f), 1730 (F), 1690 (F), 1680 (F), 1640 (F), 1540 (F), 1500 (F), 1480 (F), 1440 (F), 1400 (f), 1380 (m), 1360 (m), 1330 (m), 1310 (m), 1280 (m), 1260 (F), 1220 (F), 1200 (F), 1160 (f), 1130 (F), 1110 (F), 1060 (f), 1040 (F), 1010 (f), 1000 (f), 960 (F), 940 (m), 930 (m), 900 (m), 860 (f), 810 (m), 790 (m), 750 (F), 700 (F), 620 (F), 600 (f), 580 (f), 540 (f), 520 (f), 480 (f), 460 (f), 430 (f).

4. Procédé de préparation des formes polymorphes du (S,S)-2-(2-acétylsulfanylméthyl-3-benzo[1,3]dioxol-5-ylpropionylamino) propionate de benzyle de la revendication 1 et notamment des formes stables I ou II par cristallisation, à une température comprise entre la température de congélation de la solution et la température d'ébullition du mélange, d'une solution du (S,S) -2- (2-acétylsulfanylméthyl-3-benzo[1,3]dioxol-5ylpropionylamino)propionate de benzyle dans un solvant choisi parmi les hydrocarbures saturés ou insaturés, les alcools, les éthers, les esters, les amides, les nitriles, les halogénés, l'eau ou un mélange de ces solvants, la solution initiale de (S,S)-2-(2-acétylsulfanylméthyl-3-benzo[1,3]dioxol-5-ylpropionylamino) propionate de benzyle contenant éventuellement d'autres produits, par exemple un isomère du (S,S)-2-(2-acétylsulfanylméthyl-3-benzo[1,3]dioxol-5-ylpropionylamino) propionate de benzyle.

5. Procédé de préparation selon la revendication 4, **caractérisé en ce que** ladite cristallisation est une cristallisation spontanée ou provoquée par refroidissement lent ou rapide, ajout d'un solvant, d'amorces constituées de (S,S)-2-(2-acétylsulfanylméthyl-3-benzo[1,3]dioxol-5-ylpropionylamino)propionate de benzyle cristallisé ou d'un autre produit solide, évaporation ou distillation du solvant, la solution initiale de (S,S)-2-(2-acétylsulfanylméthyl-3-benzo[1,3]dioxol-5-ylpropionylamino) propionate de benzyle pouvant contenir d'autres produits, par exemple un isomère du (S,S)-2-(2-acétylsulfanylméthyl-3-benzo [1,3] dioxol-5-ylpropionylamino)propionate de benzyle.

6. Procédé de préparation selon l'une des revendications 4 et 5, **caractérisé en ce que** le solvant est choisi dans l'un des groupes constitués par le pentane, le cyclohexane, le toluène, le xylène, le méthanol, l'éthanol, le 2-propanol, l'oxyde de diisopropyle, l'oxyde de diéthyle, l'acétate d'éthyle, l'acétate d'isopropyle, le diméthylformamide, le N-méthylpyrrolidone, le diméthylacétamide, l'acétonitrile, le dichlorométhane, le dichloroéthane, le chlorobenzène, l'eau ou un mélange de ces solvants.

7. Procédé de préparation des formes stables I ou II du (S,S)-2-(2-acétylsulfanylméthyl-3-benzo[1,3]dioxol-5-ylpropionylamino)propionate de benzyle de l'une des revendications 1 et 2 par cristallisation d'une forme selon la revendication 1 dans une autre, en solution ou en suspension, en ensemençant éventuellement avec une forme du (S,S)-2-(2-acétylsulfanylméthyl-3-benzo[1,3]dioxol-5-ylpropionylamino)propionate de benzyle solide, à une température comprise entre la température de congélation du solvant et sa température d'ébullition, dans un solvant choisi parmi les hydrocarbures saturés ou insaturés, les alcools, les éthers, les esters, les amides, les nitriles, l'eau ou un mélange de ces solvants.

8. Procédé de préparation selon la revendication 7 **caractérisé en ce que** le milieu est choisi dans le groupe constitué par le pentane, le cyclohexane, le toluène, le xylène, le méthanol, l'éthanol, l'isopropanol, l'oxyde de diisopropyle, l'oxyde de diéthyle, l'acétate d'éthyle, l'acétate d'isopropyle, le diméthylformamide, le N-méthylpyrrolidone, le diméthylacétamide, l'acétonitrile, l'eau ou un mélange de ces solvants.

9. Compositions pharmaceutiques qui contiennent, à titre de principe actif, le (S,S)-2-(2-acétylsulfanylméthyl-3-benzo[1,3]dioxol-5-ylpropionylamino)propionate de benzyle sous l'une de ses formes polymorphes telles que définies dans la revendication 1.

10. Compositions pharmaceutiques qui contiennent, à titre de principe actif, le (S,S)-2-(2-acétylsulfanylméthyl-3-benzo[1,3] dioxol-5-ylpropionylamino) propionate de benzyle sous l'une de ses formes polymorphes stables telles que définies dans la revendication 2 ou 3.

11. Utilisation du (S,S)-2-(2-acétylsulfanylméthyl-3-benzo[1,3]dioxol-5-ylpropionylamino)propionate de benzyle sous l'une de ses formes polymorphes telles'que définies dans la revendication 1 pour la préparation d'une composition pharmaceutique pour le traitement de l'hypertension et/ou de l'insuffisance cardiaque.

12. Utilisation du (S,S)-2-(2-acétylsulfanylméthyl-3-benzo[1,3]dioxol-5-ylpropionylamino)propionate de benzyle sous l'une de ses formes polymorphes stables telles que définies dans la revendication 2 ou 3 pour la préparation d'une composition pharmaceutique pour le traitement de l'hypertension et/ou de l'insuffisance cardiaque.
